# EUROPEAN PATENT APPLICATION

(11) **EP 4 534 095 A2**
(43) Date of publication of application: **09.04.2025**
(21) Application number: 23814915.7
(22) Date of filing: 10.05.2023
(51) Int. Cl.: A61K 38/16, A61K 47/54, C07K 14/00, A61P 3/04, A61P 3/06, A61P 3/10, A61P 9/12

(54) **TREATMENT METHOD USING MAZDUTIDE**

(30) Priority: 01.06.2022 CN 202210622648
(71) Applicant: Innovent Biologics (Suzhou) Co., Ltd., Suzhou, Jiangsu 215123 (CN)
(72) Inventor: CAI, Chenghang, Suzhou, Jiangsu 215123 (CN); LIU, Meng, Suzhou, Jiangsu 215123 (CN); DENG, Huan, Suzhou, Jiangsu 215123 (CN); SONG, Baili, Suzhou, Jiangsu 215123 (CN); WEN, Jie, Suzhou, Jiangsu 215123 (CN)
(74) Representative: J A Kemp LLP
(86) International application number: PCT/CN2023/093311
(87) International publication number: WO 2023/231730

(57) **Abstract**

The present invention belongs to the field of medicine, and specifically relates to a treatment method using mazdutide. The present invention provides a method for preventing or treating overweight and obesity, and a method for improving body weight in patients in need, the method comprising administering, to a patient in need, a prophylactically or therapeutically effective amount of a target compound in a dosage regimen comprising at least three dosing cycles. The present invention also provides a target compound, which is used for preventing or treating overweight or obesity in patients in need, wherein a prophylactically or therapeutically effective amount of the target compound is administered in at least three dosing cycles to a patient in need. The mazdutide dosage regimen provided has a remarkable weight reduction effect, noticeable weight reduction can be achieved with relatively low dosage in a relatively short amount of time, and blood pressure, blood lipid, and serum uric acid levels of a subject can simultaneously be improved.

## Description

### CROSS-REFERENCE TO RELATED APPLICATIONS

The present disclosure claims priority to the invention patent application No. 202210622648.7 filed to China National Intellectual Property Administration on June 1, 2022 and entitled "TREATMENT METHOD USING MAZDUTIDE", the content of which is incorporated herein by reference in its entirety.

### TECHNICAL FIELD

The present disclosure belongs to the field of medicine, and specifically relates to a treatment method using mazdutide.

### BACKGROUND

Lifestyle interventions are the primary treatment for overweight or obese individuals, but many patients are unable to achieve weight loss or fail to reach their desired weight loss goals for various reasons. For such patients, medication-assisted weight loss may be considered as an option. According to China's guidelines, pharmacological weight loss treatment is recommended in addition to lifestyle and behavioral interventions for patients with a BMI of 28 kg/m² or higher who fail to achieve a 5% weight loss after three months of lifestyle interventions, or for patients with a BMI of 24 kg/m² or higher who have obesity-related complications such as hyperglycemia, hypertension, dyslipidemia, non-alcoholic fatty liver disease (NAFLD), weight-bearing joint pain, or sleep apnea syndrome.

Currently, FDA approved long-term (≥ 12 weeks) obesity treatment drugs mainly include: phentermine and topiramate sustained-release capsules, bupropion hydrochloride-naltrexone hydrochloride, liraglutide and semaglutide injections, and weight loss drugs for short-term use (< 12 weeks) including amfepramone (diethylpropion), phendimetrazine, phentermine, benzphetamine, and the like. Early weight loss drugs, while effective in reducing body weight to some extent, also had concerning side effects that limited their use. For example, amfepramone may cause tachycardia, insomnia, and the like; phentermine may cause insomnia, dry mouth, and constipation; the combination of phentermine and fenfluramine may induce primary pulmonary hypertension and cardiac valve insufficiency, although phentermine alone has not shown a significant correlation with these conditions; orlistat may cause fat-soluble vitamin deficiency, gastrointestinal flatulence, fecal urgency, fatty diarrhea, and the like. Various GLP-1 single receptor agonists are currently on the market, such as liraglutide. Taking semaglutide as an example, it is one of the most effective weight loss medications available globally. With administration for 12 consecutive weeks, it can result in approximately a 6% weight reduction, and with continuous use for a year, the weight loss can reach about 13-16%. Obese people with higher BMIs, such as those with a BMI greater than 30 kg/m², have a greater need for weight loss. Although GLP-1 single receptor agonists have demonstrated some efficacy in weight loss, there is still a significant unmet medical need in the treatment of obesity.

In recent years, multiple receptor agonists targeting multiple metabolic pathways have become a hotspot for drug development in the field of metabolic diseases. Results from a number of preclinical and clinical studies have shown that GLP-1 receptor agonists exhibit an additive or synergistic effect when co-administered with other incretins. Compared to single-target agonists used alone or in combination, single-molecule multi-receptor agonists with balanced activity across multiple receptors hold promise for further improvements in maximizing efficacy, reducing side effects, and enhancing pharmacokinetics. Thus, GLP-1-based unimolecular multiple receptor agonists have the benefits of synergistic effect, tolerability improvement, safety improvement, etc.

Mazdutide is a synthetic long-acting peptide analog to mammalian oxyntomodulin (OXM). OXM is a peptide hormone secreted by human intestinal L cells following nutritional ingestion and is a dual agonist of the glucagon-like peptide-1 receptor (GLP-1R) and the glucagon receptor (GCGR). The GLP-1R and GCGR dual agonist combines the thermogenesis and lipolysis effects of glucagon with the gastric emptying delaying effect of GLP-1 to generate a remarkable weight loss effect, and buffers the blood glucose increasing effect of the glucagon through the insulin secretion promoting effect of GLP-1, thus effectively controlling the blood glucose. OXM injection in humans can significantly reduce body weight and appetite, and increase energy expenditure. As an OXM analog, mazdutide works by activating GLP-1R and GCGR simultaneously.

### SUMMARY

### Problems to be solved herein

The present disclosure is intended to provide a mazdutide dosing regimen that will fully deliver the therapeutic effect of mazdutide on obesity or overweight with the proper amount of drug and at the right time.

### Solutions for solving the problems

[1] A method for preventing or treating obesity or overweight, comprising administering to a patient in need a prophylactically or therapeutically effective amount of a target compound in an dosing regimen consisting of at least three dosing cycles, wherein the at least three dosing cycles comprise:
   (i) a first dosing cycle for at least 3 weeks of administering about one unit dose of the target compound weekly;
   (ii) a second dosing cycle for at least 3 weeks of administering about two unit doses of the target compound weekly;
   (iii) a third dosing cycle for at least 3 weeks of administering about three unit doses of the target compound weekly; and optionally,
   (iv) a fourth dosing cycle for at least 3 weeks of administering about four unit doses of the target compound weekly,
   wherein the unit dose is 2.2 mg-3.5 mg, and the target compound is selected from mazdutide or a pharmaceutically acceptable salt thereof.
[2] The method according to [1], wherein the duration of the last dosing cycle in the dosing regimen is at least 3 weeks, preferably at least 4 weeks; the duration of each of the remaining dosing cycles is independently 3 weeks-5 weeks, preferably about 4 weeks;
   optionally,
   the third dosing cycle is taken as the last dosing cycle in the dosing regimen; moreover, the duration of the third dosing cycle is at least 3 weeks, preferably at least 4 weeks;
   alternatively,
   the fourth dosing cycle is taken as the last dosing cycle in the dosing regimen; moreover, the duration of the fourth dosing cycle is at least 3 weeks, preferably at least 4 weeks.
[3] The method according to [1] or [2], wherein in each of the dosing cycles, the target compound is administered to the patient one or more times weekly;
   preferably, in each of the dosing cycles, the target compound is administered to the patient once weekly.
[4] The method according to any one of [1]-[3], wherein the unit dose is 2.2 mg-3 mg, 2.2 mg-2.8 mg, 2.4 mg-2.6 mg, 2.5 mg-3.5 mg, 2.7 mg-3.3 mg, or 2.9 mg-3.1 mg;
   optionally, the unit dose is about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, or about 3.5 mg.
[5] The method according to any one of [1]-[4], comprising administering the target compound in the dosing regimen I as shown below:
   administering to the patient 2.2 mg-3 mg of the target compound weekly for 3-5 weeks in the first dosing cycle;
   subsequently, administering to the patient 4.5 mg-5.5 mg of the target compound weekly for 3-5 weeks in the second dosing cycle;
   subsequently, administering to the patient 7 mg-8 mg of the target compound weekly for 3-5 weeks in the third dosing cycle;
   subsequently, administering to the patient 9.5 mg-10.5 mg of the target compound weekly for at least 3 weeks in the fourth dosing cycle;
   optionally, in the first dosing cycle, administering to the patient 2.2 mg-2.8 mg of the target compound weekly for about 4 weeks; preferably administering to the patient about 2.5 mg of the target compound weekly for about 4 weeks; more preferably administering to the patient about 2.5 mg of the target compound once weekly for about 4 weeks;
   optionally, in the second dosing cycle, administering to the patient 4.7 mg-5.3 mg of the target compound weekly for about 4 weeks; preferably administering to the patient about 5 mg of the target compound weekly for about 4 weeks; more preferably administering to the patient about 5 mg of the target compound once weekly for about 4 weeks;
   optionally, in the third dosing cycle, administering to the patient 7.2 mg-7.8 mg of the target compound weekly for about 4 weeks; preferably administering to the patient about 7.5 mg of the target compound weekly for about 4 weeks; more preferably administering to the patient about 7.5 mg of the target compound once weekly for about 4 weeks;
   optionally, in the fourth dosing cycle, administering to the patient 9.7 mg-10.3 mg of the target compound weekly for at least 4 weeks; preferably administering to the patient about 10 mg of the target compound weekly for at least 4 weeks; more preferably administering to the patient about 10 mg of the target compound once weekly for at least 4 weeks.
[6] The method according to any one of [1]-[4], comprising administering the target compound in the dosing regimen II as shown below:
   administering to the patient 2.5 mg-3.5 mg of the target compound weekly for 3-5 weeks in the first dosing cycle;
   subsequently, administering to the patient 5.5 mg-6.5 mg of the target compound weekly for 3-5 weeks in the second dosing cycle;
   subsequently, administering to the patient 8.5 mg-9.5 mg of the target compound weekly for at least 3 weeks in the third dosing cycle;
   optionally, in the first dosing cycle, administering to the patient 2.7 mg-3.3 mg of the target compound weekly for about 4 weeks; preferably administering to the patient about 3 mg of the target compound weekly for about 4 weeks; more preferably administering to the patient about 3 mg of the target compound once weekly for about 4 weeks;
   optionally, in the second dosing cycle, administering to the patient 5.7 mg-6.3 mg of the target compound weekly for about 4 weeks; preferably administering to the patient about 6 mg of the target compound weekly for about 4 weeks; more preferably administering to the patient about 6 mg of the target compound once weekly for about 4 weeks;
   optionally, in the third dosing cycle, administering to the patient 8.7 mg-9.3 mg of the target compound weekly for at least 4 weeks; preferably administering to the patient about 9 mg of the target compound weekly for at least 4 weeks; more preferably administering to the patient about 9 mg of the target compound once weekly for at least 4 weeks;
   optionally, in the third dosing cycle, administering to the patient 8.7 mg-9.3 mg of the target compound weekly for at least 16 weeks; preferably administering to the patient about 9 mg of the target compound weekly for at least 16 weeks; more preferably administering to the patient about 9 mg of the target compound once weekly for at least 16 weeks;
   optionally, in the third dosing cycle, administering to the patient 8.7 mg-9.3 mg of the target compound weekly for at least 40 weeks; preferably administering to the patient about 9 mg of the target compound weekly for at least 40 weeks; more preferably administering to the patient about 9 mg of the target compound once weekly for at least 40 weeks.
[7] The method according to any one of [1]-[6], wherein the administration is by injection, preferably subcutaneous injection.
[8] A method for improving body weight in a patient in need, comprising administering to the patient a prophylactically or therapeutically effective amount of a target compound in an dosing regimen consisting of at least three dosing cycles, wherein the at least three dosing cycles comprise:
   (i) a first dosing cycle for at least 3 weeks of administering about one unit dose of the target compound weekly;
   (ii) a second dosing cycle for at least 3 weeks of administering about two unit doses of the target compound weekly;
   (iii) a third dosing cycle for at least 3 weeks of administering about three unit doses of the target compound weekly; and optionally,
   (iv) a fourth dosing cycle for at least 3 weeks of administering about four unit doses of the target compound weekly,

   wherein the unit dose is 2.2 mg-3.5 mg, and the target compound is selected from mazdutide or a pharmaceutically acceptable salt thereof;
   preferably, a prophylactically or therapeutically effective amount of the target compound is administered to the patient using the method according to any one of [1]-[7].
[9] A target compound for use in preventing or treating obesity or overweight in a patient in need, wherein a prophylactically or therapeutically effective amount of the target compound is administered to a patient in need in an dosing regimen consisting of at least three dosing cycles; the at least three dosing cycles comprise:
   (i) a first dosing cycle for at least 3 weeks of administering about one unit dose of the target compound weekly;
   (ii) a second dosing cycle for at least 3 weeks of administering about two unit doses of the target compound weekly;
   (iii) a third dosing cycle for at least 3 weeks of administering about three unit doses of the target compound weekly; and optionally,
   (iv) a fourth dosing cycle for at least 3 weeks of administering about four unit doses of the target compound weekly,

   wherein the unit dose is 2.2 mg-3.5 mg, and the target compound is selected from mazdutide or a pharmaceutically acceptable salt thereof;
   preferably, a prophylactically or therapeutically effective amount of the target compound is administered to the patient using the method according to any one of [1]-[7].

### Beneficial effects

In some embodiments, the method for preventing or treating obesity or overweight provided by the present disclosure can achieve a significant weight loss effect and improve blood pressure, blood lipid, and serum uric acid levels in a subject by administering mazdutide or a pharmaceutically acceptable salt thereof at a specific dosing regimen.

In some specific embodiments, the present disclosure administers to a patient in need mazdutide or a pharmaceutically acceptable salt thereof using dosing regimen I or dosing regimen II with different administration doses. The present disclosure finds that both dosing regimen I and dosing regimen II, with different mazdutide dosages, have remarkable weight loss efficacy, and patients exhibit good overall tolerability and safety during the administration process, indicating a high safety of both dosing regimens for clinical use.

In some preferred embodiments, the present disclosure administers to a patient in need mazdutide or a pharmaceutically acceptable salt thereof using dosing regimen II. Dosing regimen II has a more superior weight loss effect than dosing regimen I. At the same time, the present disclosure surprisingly discovered that compared to dosing regimen I, dosing regimen II, which involves a shorter duration and lower dose of mazdutide, results in greater weight loss in subjects. Therefore, the dosing regimen II provided by the present disclosure does not rely on increasing doses and duration for better weight loss effects; instead, it optimizes weight reduction with lower doses and shorter time, showing significant promise for clinical application.

In addition, the dosing regimen II provided by the present disclosure has more prominent weight loss effect compared with the dosing regimens in which other similar drugs are administered.

Meanwhile, the present disclosure further obtains better weight loss efficacy by prolonging the administration time of the maintenance dose in dosing regimen II, and the patients also have good overall tolerability and safety in the prolonged administration process, which has high clinical administration safety.

### BRIEF DESCRIPTION OF THE DRAWINGS

FIG. 1 is the percent change results from baseline in body weight of the subjects for dosing regimens of cohorts 1-5.
FIG. 2 is the mean change results from baseline in BMI of the subjects for dosing regimens of cohorts 4-5.
FIG. 3 is the mean change results from baseline in blood pressure of the subjects for dosing regimens of cohorts 4-5.
FIG. 4 is the mean change results from baseline in blood lipid of the subjects for dosing regimens of cohorts 4-5.
FIG. 5 is the mean change results from baseline in serum uric acid level of the subjects for dosing regimens of cohorts 4-5.
FIG. 6 is the indirect comparison results of the percent change from baseline in body weight of the subjects at week 12 for dosing regimens of mazdutide cohorts 2, 3, and 5 and dosing regimens of similar drugs at different doses.

### DETAILED DESCRIPTION

The embodiments of the present disclosure will be described below, but the present disclosure is not limited thereto.

In the present disclosure, the terms "a" or "an" or "the" may mean "one", and may also mean "one or more", "at least one", and "one or more than one".

In the present disclosure, the terms "comprise", "have", "include", or "contain" may be intended to be inclusive or open-ended and do not exclude additional and unreferenced elements or method steps. Also, "comprise", "have", "include", or "contain" may also be intended to be close-ended and exclude additional and unreferenced elements or method steps.

In the present disclosure, the meaning of "may" includes both the meaning of performing a certain process and the meaning of not performing a certain process.

In the present disclosure, "optional" or "optionally" means that the subsequently described event or circumstance may or may not occur, and that the description includes instances where the event occurs and instances where it does not.

In the present disclosure, the numerical ranges indicated by the use of "at least numerical value A", "at most numerical value A", "numerical value A to numerical value B", or "numerical value A-numerical value B" refer to ranges including the endpoint numerical values A and B.

In the present disclosure, "about" is used to define the numerical ranges and parameters of the present disclosure, which are approximations, and the specific related numerical values are presented herein as precisely as possible. Unless otherwise specifically stated, it should be understood that all ranges, amounts, values and percentages used herein are modified by "about". As used herein, "about" generally means that the actual value is within ±10%, ±5%, ±1%, or ±0.5% of a particular value or range.

In the present disclosure, the terms "polypeptide", "peptide", and "protein" are used interchangeably herein and are amino acid polymers of any length. The polymer can be linear or branched, can comprise modified amino acids, and can be interrupted by non-amino acids. The term also includes amino acid polymers which have been modified (e.g., formation of disulfide bond, glycosylation, lipidation, acetylation, phosphorylation, or any other operation such as conjugation with a labeling component).

In the present disclosure, "unit dose" refers to the initial dose of a drug that is given to a patient in each dosing regimen, which is generally less than the maximum effective dose required by the patient.

In the present disclosure, the "maintenance dose" refers to the maximum drug dose that is given to a patient in each dosing regimen, i.e., the maximum effective dose required by the patient, and the maintenance dose may be administered for a period of time exceeding 3 weeks.

In the present disclosure, "treatment" includes delaying or attenuating the progression of a disease or disorder, including alleviating, relieving, or reducing one or more symptoms of the disorder or condition, and does not mean that the symptoms of the disease must be completely inhibited. In some embodiments, the "treating obesity or overweight" described herein includes delaying body weight gain, reducing body weight, improving blood pressure, improving blood lipid, improving the serum urine level, and the like. In some embodiments, improving blood pressure described herein includes, but is not limited to, reducing the diastolic and systolic blood pressure. In some embodiments, improving blood lipid described herein includes, but is not limited to, reducing the content of low-density lipoprotein cholesterol, reducing the content of triglyceride, and/or reducing the content of total cholesterol. In some embodiments, improving the serum urine level described herein includes, but is not limited to, reducing the content of serum uric acid.

In the present disclosure, the term "prevention" means: by contacting (e.g., administering) the compound of the present disclosure or a pharmaceutically acceptable salt thereof or the pharmaceutical composition of the present disclosure to a subject before the onset of a disease, the symptoms after the onset of the disease are alleviated as compared to when not contacted, and it is not necessarily required to completely inhibit the onset of the disease.

In the present disclosure, the term "improving body weight" refers to a decrease in body weight of a subject. In the present disclosure, "obesity" means BMI ≥ 28.0 kg/m².

In the present disclosure, "overweight" means 24 kg/m² ≤ BMI < 28.0 kg/m² with at least one of the following: i, increased appetite, intolerable hunger before meals, and increased food consumption; ii, comorbidity with one or more of pre-diabetes (impaired fasting glucose and/or impaired glucose tolerance), hypertension, dyslipidemia, and fatty liver (within 6 months before the screening); iii, comorbidity with weight-bearing joint pain; iv, obesity-related dyspnea or obstructive sleep apnea syndrome.

In the present disclosure, the term "prophylactically or therapeutically effective amount" refers to an amount effective to achieve a desired prophylactic or therapeutic result at a necessary dose for a necessary period of time.

The compound of the present disclosure may also be provided in the form of a salt. These salts may be formed using commonly performed methods.

In the present disclosure, the term "pharmaceutically acceptable salt" refers to a salt prepared from a target compound with a relatively nontoxic acid or base. When the target compound contains a relatively acidic functional group (e.g., a carboxyl group or a sulfonic acid group), a base addition salt can be obtained by contacting the free form thereof with a sufficient amount of a base in a pure solution or a suitable inert solvent. Non-limiting examples of pharmaceutically acceptable base addition salts include, but are not limited to, sodium salts, potassium salts, ammonium salts, calcium salts, magnesium salts, organic amine salts, or similar salts. When the compound of the present disclosure contains a relatively basic functional group (e.g., an amino group or a guanidino group), an acid addition salt can be obtained by contacting the free form thereof with a sufficient amount of an acid in a pure solution or a suitable inert solvent. Non-limiting examples of pharmaceutically acceptable acid addition salts include, but are not limited to, inorganic acid salts (e.g., hydrochloride, hydrobromide, hydroiodide, nitrate, carbonate, bicarbonate, phosphate, monohydrogen phosphate, dihydrogen phosphate, phosphite, sulfate, bisulfate, and the like), organic acid salts (e.g., acetate, propionate, isobutyrate, malonate, succinate, suberate, maleate, fumarate, citrate, tartrate, lactate, mandelate, benzoate, phthalate, methanesulfonate, benzenesulfonate, p-toluenesulfonate, glucuronic acid, and the like), and amino acid salts (e.g., arginine salts and the like). Specific forms of pharmaceutically acceptable salts can also be found in Berge et al., "Pharmaceutical Salts", Journal of Pharmaceutical Science, 1977, 66: 1-19).

In the present disclosure, "administration" means administration by a nurse, health care provider, patient, or any other individual, including self-administration. The administration includes not only delivery into the body, but also prescribing, distributing, or assisting in delivery in any way.

In the present disclosure, "individual", "patient", or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the patient described herein includes an obese and/or overweight person.

### Mazdutide or pharmaceutically acceptable salt thereof

The mazdutide described herein is a dual agonist for glucagon-like peptide-1 (GLP-1) and glucagon receptor (GCGR), and can bind to and activate GLP-1R and GCGR. Mazdutide may refer to that shown in patent CN201680036771.3, the content of which is incorporated herein by reference in its entirety.

Mazdutide has the sequence set forth in SEQ ID NO. 1:
His-Xaa-Gln-Gly-Thr-Phe-Thr-Ser-Asp-Tyr-Ser-Lys-Tyr-Leu-Asp-Glu-Lys-Lys-Ala-Lys-Glu-Phe-Val-Glu-Trp-Leu-Leu-Glu-Gly-Gly-Pro-Ser-Ser-Gly, wherein Xaa is Aib; the Lys at position 20 is chemically modified by conjugating with ε-amino group of the side chain via ([2-(2-amino-ethoxy)-ethoxy]-acetyl)₂-(γGlu)₁-CO-(CH₂)₁₈-CO₂H, and the carboxyl group of the C-terminal Gly was amidated to a C-terminal primary amide.

The chemical formula of mazdutide is shown below:

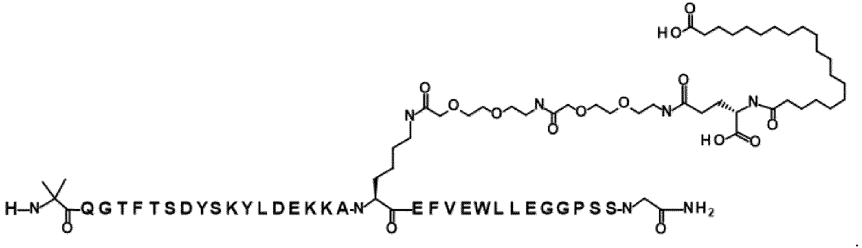

The mazdutide of the present disclosure can be reacted with any number of inorganic and organic acids to form pharmaceutically acceptable acid addition salts. Pharmaceutically acceptable salts and conventional methodologies for preparing them are well known in the art. See, e.g., P. Stahl et al., Handbook of Pharmaceutical Salts: Properties, Selection and Use, 2nd revised edition (Wiley-VCH, 2011). Pharmaceutically acceptable salts of mazdutide of the present disclosure include trifluoroacetate, hydrochloride, and acetate.

### Medical use and dosing regimen

The mazdutide or the pharmaceutically acceptable salt thereof described herein can be used for preventing or treating obesity or overweight, and further, administering the mazdutide or the pharmaceutically acceptable salt thereof at a specific dosing regimen can effectively reduce the body weight, and improve the blood pressure, the blood lipid, the serum uric acid level, and the like in an obese patient or an overweight patient.

### <First aspect>

The present disclosure provides a method for preventing or treating obesity or overweight, comprising administering to a patient in need a prophylactically or therapeutically effective amount of a target compound in an dosing regimen consisting of at least three dosing cycles, wherein the at least three dosing cycles comprise:
(i) a first dosing cycle for at least 3 weeks of administering about one unit dose of the target compound weekly;
(ii) a second dosing cycle for at least 3 weeks of administering about two unit doses of the target compound weekly;
(iii) a third dosing cycle for at least 3 weeks of administering about three unit doses of the target compound weekly; and optionally,
(iv) a fourth dosing cycle for at least 3 weeks of administering about four unit doses of the target compound weekly,
wherein the unit dose is 2.2 mg-3.5 mg, and the target compound is selected from mazdutide or a pharmaceutically acceptable salt thereof.

In some embodiments, the method for preventing or treating obesity or overweight described herein comprises administering to a patient in need a prophylactically or therapeutically effective amount of a target compound in an dosing regimen consisting of three dosing cycles, and the three dosing cycles are:
(i) a first dosing cycle for at least 3 weeks of administering about one unit dose of the target compound weekly;
(ii) a second dosing cycle for at least 3 weeks of administering about two unit doses of the target compound weekly;
(iii) a third dosing cycle for at least 3 weeks of administering about three unit doses of the target compound weekly,
wherein the unit dose is 2.2 mg-3.5 mg, and the target compound is selected from mazdutide or a pharmaceutically acceptable salt thereof.

In some other embodiments, the method for preventing or treating obesity or overweight described herein comprises administering to a patient in need a prophylactically or therapeutically effective amount of a target compound in an dosing regimen consisting of four dosing cycles, and the four dosing cycles are:
(i) a first dosing cycle for at least 3 weeks of administering about one unit dose of the target compound weekly;
(ii) a second dosing cycle for at least 3 weeks of administering about two unit doses of the target compound weekly;
(iii) a third dosing cycle for at least 3 weeks of administering about three unit doses of the target compound weekly,
(iv) a fourth dosing cycle for at least 3 weeks of administering about four unit doses of the target compound weekly,
wherein the unit dose is 2.2 mg-3.5 mg, and the target compound is selected from mazdutide or a pharmaceutically acceptable salt thereof.

### (Duration of dosing cycle)

Each of the dosing cycles described herein will vary in duration due to chronological order and the maximum drug dose required by the patient.

In some embodiments, the duration of each of the dosing cycles described herein is independently at least 3 weeks.

Further, in some embodiments, in the dosing regimen of the present disclosure, the duration of the last dosing cycle is at least 3 weeks, preferably at least 4 weeks; the duration of each of the remaining dosing cycles is independently 3 weeks-5 weeks, preferably 4 weeks.

In the present disclosure, when the third dosing cycle is taken as the last dosing cycle of the dosing regimen, the duration of the third dosing cycle is at least 3 weeks, preferably at least 4 weeks. Illustratively, the duration of the third dosing cycle is at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, at least 15 weeks, at least 16 weeks, at least 20 weeks, at least 30 weeks, at least 40 weeks, and the like. The present disclosure is not exhaustive of the duration of the third dosing cycle which is taken as the last dosing cycle. The duration of the third dosing cycle may be maintained until such time as the desired prophylactic or therapeutic result is achieved or the treatment is concluded as determined by a physician or other health care providers.

In the present disclosure, when the fourth dosing cycle is taken as the last dosing cycle of the dosing regimen, the duration of the fourth dosing cycle is at least 3 weeks, preferably at least 4 weeks. Illustratively, the duration of the fourth dosing cycle is at least 3 weeks, at least 4 weeks, at least 5 weeks, at least 6 weeks, at least 7 weeks, at least 8 weeks, at least 9 weeks, at least 10 weeks, at least 11 weeks, at least 12 weeks, at least 13 weeks, at least 14 weeks, at least 15 weeks, at least 20 weeks, at least 30 weeks, and the like. The present disclosure is not exhaustive of the duration of the fourth dosing cycle which is taken as the last dosing cycle. The duration of the fourth dosing cycle may be maintained until such time as the desired prophylactic or therapeutic result is achieved or the treatment is concluded as determined by a physician or other health care providers.

In some preferred embodiments, the method for preventing or treating obesity or overweight described herein comprises administering to a patient in need a prophylactically or therapeutically effective amount of a target compound in an dosing regimen consisting of three dosing cycles, and the three dosing cycles are:
(i) a first dosing cycle for 3-5 weeks of administering about one unit dose of the target compound weekly;
(ii) a second dosing cycle for 3-5 weeks of administering about two unit doses of the target compound weekly;
(iii) a third dosing cycle for at least 3 weeks of administering about three unit doses of the target compound weekly,
wherein the unit dose is 2.2 mg-3.5 mg, and the target compound is selected from mazdutide or a pharmaceutically acceptable salt thereof. At this time, in this embodiment, the dose administered in the third dosing cycle is a maintenance dose.

In some more preferred embodiments, the method for preventing or treating obesity or overweight described herein comprises administering to a patient in need a prophylactically or therapeutically effective amount of a target compound in an dosing regimen consisting of three dosing cycles, and the three dosing cycles are:
(i) a first dosing cycle for about 4 weeks of administering about one unit dose of the target compound weekly;
(ii) a second dosing cycle for about 4 weeks of administering about two unit doses of the target compound weekly;
(iii) a third dosing cycle for at least 4 weeks of administering about three unit doses of the target compound weekly,
wherein the unit dose is 2.2 mg-3.5 mg, and the target compound is selected from mazdutide or a pharmaceutically acceptable salt thereof. At this time, in this embodiment, the dose administered in the third dosing cycle is a maintenance dose.

In some other more preferred embodiments, the method for preventing or treating obesity or overweight described herein comprises administering to a patient in need a prophylactically or therapeutically effective amount of a target compound in an dosing regimen consisting of three dosing cycles, and the three dosing cycles are:
(i) a first dosing cycle for about 4 weeks of administering about one unit dose of the target compound weekly;
(ii) a second dosing cycle for about 4 weeks of administering about two unit doses of the target compound weekly;
(iii) a third dosing cycle for at least 16 weeks of administering about three unit doses of the target compound weekly,
wherein the unit dose is 2.2 mg-3.5 mg, and the target compound is selected from mazdutide or a pharmaceutically acceptable salt thereof. At this time, in this embodiment, the dose administered in the third dosing cycle is a maintenance dose.

In some other more preferred embodiments, the method for preventing or treating obesity or overweight described herein comprises administering to a patient in need a prophylactically or therapeutically effective amount of a target compound in an dosing regimen consisting of three dosing cycles, and the three dosing cycles are:
(i) a first dosing cycle for about 4 weeks of administering about one unit dose of the target compound weekly;
(ii) a second dosing cycle for about 4 weeks of administering about two unit doses of the target compound weekly;
(iii) a third dosing cycle for at least 40 weeks of administering about three unit doses of the target compound weekly,
wherein the unit dose is 2.2 mg-3.5 mg, and the target compound is selected from mazdutide or a pharmaceutically acceptable salt thereof. At this time, in this embodiment, the dose administered in the third dosing cycle is a maintenance dose.

In some other preferred embodiments, the method for preventing or treating obesity or overweight described herein comprises administering to a patient in need a prophylactically or therapeutically effective amount of a target compound in an dosing regimen consisting of four dosing cycles, and the four dosing cycles are:
(i) a first dosing cycle for 3-5 weeks of administering about one unit dose of the target compound weekly;
(ii) a second dosing cycle for 3-5 weeks of administering about two unit doses of the target compound weekly;
(iii) a third dosing cycle for 3-5 weeks of administering about three unit doses of the target compound weekly;
(iv) a fourth dosing cycle for at least 3 weeks of administering about four unit doses of the target compound weekly,
wherein the unit dose is 2.2 mg-3.5 mg, and the target compound is selected from mazdutide or a pharmaceutically acceptable salt thereof. At this time, in this embodiment, the dose administered in the fourth dosing cycle is a maintenance dose.

In some other more preferred embodiments, the method for preventing or treating obesity or overweight described herein comprises administering to a patient in need a prophylactically or therapeutically effective amount of a target compound in an dosing regimen consisting of four dosing cycles, and the four dosing cycles are:
(i) a first dosing cycle for about 4 weeks of administering about one unit dose of the target compound weekly;
(ii) a second dosing cycle for about 4 weeks of administering about two unit doses of the target compound weekly;
(iii) a third dosing cycle for about 4 weeks of administering about three unit doses of the target compound weekly;
(iv) a fourth dosing cycle for at least 4 weeks of administering about four unit doses of the target compound weekly,
wherein the unit dose is 2.2 mg-3.5 mg, and the target compound is selected from mazdutide or a pharmaceutically acceptable salt thereof. At this time, in this embodiment, the dose administered in the fourth dosing cycle is a maintenance dose.

### (Administration frequency)

The number of administrations per dosing cycle in the present disclosure is not particularly limited as long as a sufficient dose of the drug of the required amount in each dosing cycle is administered. In some embodiments, in each of the dosing cycles, the target compound is administered to the patient one or more times weekly. In some embodiments, in each of the dosing cycles, the target compound is administered to the patient once weekly.

In some preferred embodiments, the method for preventing or treating obesity or overweight described herein comprises administering to a patient in need a prophylactically or therapeutically effective amount of a target compound in an dosing regimen consisting of at least three dosing cycles, wherein the dosing regimen comprises:
(i) a first dosing cycle for at least 3 weeks of administering about one unit dose of the target compound once weekly;
(ii) a second dosing cycle for at least 3 weeks of administering about two unit doses of the target compound once weekly;
(iii) a third dosing cycle for at least 3 weeks of administering about three unit doses of the target compound once weekly; and optionally,
(iv) a fourth dosing cycle for at least 3 weeks of administering about four unit doses of the target compound once weekly,
wherein the unit dose is 2.2 mg-3.5 mg, and the target compound is selected from mazdutide or a pharmaceutically acceptable salt thereof.

### (Dose of administration)

In the method for preventing or treating obesity or overweight described herein, the administration dose is increased approximately several times as the dosing cycle advances. In each dosing regimen, the initial dose administered to the patient is a unit dose, which is increased as the dosing cycle progresses until the patient is given the maximum effective dose required, i.e., the maintenance dose, and then the administration dose is no longer increased.

In some embodiments, the unit dose described herein is 2.2 mg-3.5 mg.

In some embodiments, the unit dose described herein is 2.2 mg-3 mg, 2.2 mg-2.8 mg, 2.4 mg-2.6 mg, 2.5 mg-3.5 mg, 2.7 mg-3.3 mg, 2.9 mg-3.1 mg, or the like.

In some more specific embodiments, the unit dose described herein is about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, about 3.5 mg, or the like.

### (Dosing regimen)

In the method for preventing or treating obesity or overweight of the present disclosure, there may be a variety of different target compound dosing regimens, all of which can achieve the effects of reducing body weight, improving blood pressure, improving blood lipid, improving serum urine levels, etc., in a subject.

In some embodiments, the method for preventing or treating obesity or overweight described herein comprises administering a target compound in the dosing regimen as shown below:
administering to the patient about one unit dose of the target compound weekly for at least 3 weeks in the first dosing cycle;
subsequently, administering to the patient about two unit doses of the target compound weekly for at least 3 weeks in the second dosing cycle;
subsequently, administering to the patient about three unit doses of the target compound weekly for at least 3 weeks in the third dosing cycle;
alternatively,
administering to the patient about one unit dose of the target compound weekly for at least 3 weeks in the first dosing cycle;
subsequently, administering to the patient about two unit doses of the target compound weekly for at least 3 weeks in the second dosing cycle;
subsequently, administering to the patient about three unit doses of the target compound weekly for at least 3 weeks in the third dosing cycle;
subsequently, administering to the patient about four unit doses of the target compound weekly for at least 3 weeks in the fourth dosing cycle.

### Dosing regimen I

In some specific embodiments, the method for preventing or treating obesity or overweight described herein comprises administering a target compound in the dosing regimen I as shown below:
administering to the patient 2.2 mg-3 mg of the target compound weekly for 3-5 weeks in the first dosing cycle;
subsequently, administering to the patient 4.5 mg-5.5 mg of the target compound weekly for 3-5 weeks in the second dosing cycle;
subsequently, administering to the patient 7 mg-8 mg of the target compound weekly for 3-5 weeks in the third dosing cycle;
subsequently, administering to the patient 9.5 mg-10.5 mg of the target compound weekly for at least 3 weeks in the fourth dosing cycle.

Further, in some specific embodiments, the method for preventing or treating obesity or overweight described herein comprises administering a target compound in the dosing regimen I as shown below:
administering to the patient 2.2 mg-2.8 mg of the target compound weekly for about 4 weeks in the first dosing cycle;
subsequently, administering to the patient 4.7 mg-5.3 mg of the target compound weekly for about 4 weeks in the second dosing cycle;
subsequently, administering to the patient 7.2 mg-7.8 mg of the target compound weekly for about 4 weeks in the third dosing cycle;
subsequently, administering to the patient 9.7 mg-10.3 mg of the target compound weekly for at least 4 weeks in the fourth dosing cycle.

Further still, in some specific embodiments, the method for preventing or treating obesity or overweight described herein comprises administering a target compound in the dosing regimen I as shown below:
administering to the patient 2.5 mg of the target compound weekly for about 4 weeks in the first dosing cycle; subsequently, administering to the patient 5 mg of the target compound weekly for about 4 weeks in the second dosing cycle;
subsequently, administering to the patient 7.5 mg of the target compound weekly for about 4 weeks in the third dosing cycle;
subsequently, administering to the patient 10 mg of the target compound weekly for at least 4 weeks in the fourth dosing cycle.

Even further, in some specific embodiments, the method for preventing or treating obesity or overweight described herein comprises administering a target compound in the dosing regimen I as shown below:
administering to the patient 2.5 mg of the target compound once weekly for about 4 weeks in the first dosing cycle;
subsequently, administering to the patient 5 mg of the target compound once weekly for about 4 weeks in the second dosing cycle;
subsequently, administering to the patient 7.5 mg of the target compound once weekly for about 4 weeks in the third dosing cycle;
subsequently, administering to the patient 10 mg of the target compound once weekly for at least 4 weeks in the fourth dosing cycle.

### Dosing regimen II

In some specific embodiments, the method for preventing or treating obesity or overweight described herein comprises administering a target compound in the dosing regimen II as shown below:
administering to the patient 2.5 mg-3.5 mg of the target compound weekly for 3-5 weeks in the first dosing cycle;
subsequently, administering to the patient 5.5 mg-6.5 mg of the target compound weekly for 3-5 weeks in the second dosing cycle;
subsequently, administering to the patient 8.5 mg-9.5 mg of the target compound weekly for at least 3 weeks in the third dosing cycle.

Further, in some specific embodiments, the method for preventing or treating obesity or overweight described herein comprises administering a target compound in the dosing regimen II as shown below:
administering to the patient 2.7 mg-3.3 mg of the target compound weekly for about 4 weeks in the first dosing cycle;
subsequently, administering to the patient 5.7 mg-6.3 mg of the target compound weekly for about 4 weeks in the second dosing cycle;
subsequently, administering to the patient 8.7 mg-9.3 mg of the target compound weekly for at least 4 weeks in the third dosing cycle.

Further still, in some specific embodiments, the method for preventing or treating obesity or overweight described herein comprises administering a target compound in the dosing regimen II as shown below:
administering to the patient 3 mg of the target compound weekly for about 4 weeks in the first dosing cycle;
subsequently, administering to the patient 6 mg of the target compound weekly for about 4 weeks in the second dosing cycle;
subsequently, administering to the patient 9 mg of the target compound weekly for at least 4 weeks in the third dosing cycle.

Even further, in some specific embodiments, the method for preventing or treating obesity or overweight described herein comprises administering a target compound in the dosing regimen II as shown below:
administering to the patient 3 mg of the target compound once weekly for about 4 weeks in the first dosing cycle;
subsequently, administering to the patient 6 mg of the target compound once weekly for about 4 weeks in the second dosing cycle;
subsequently, administering to the patient 9 mg of the target compound once weekly for at least 4 weeks in the third dosing cycle.

In some other specific embodiments, the method for preventing or treating obesity or overweight described herein comprises administering a target compound in the dosing regimen II as shown below:
administering to the patient 2.5 mg-3.5 mg of the target compound weekly for 3-5 weeks in the first dosing cycle;
subsequently, administering to the patient 5.5 mg-6.5 mg of the target compound weekly for 3-5 weeks in the second dosing cycle;
subsequently, administering to the patient 8.5 mg-9.5 mg of the target compound weekly for at least 16 weeks in the third dosing cycle.

Further, in some other specific embodiments, the method for preventing or treating obesity or overweight described herein comprises administering a target compound in the dosing regimen II as shown below:
administering to the patient 2.7 mg-3.3 mg of the target compound weekly for about 4 weeks in the first dosing cycle;
subsequently, administering to the patient 5.7 mg-6.3 mg of the target compound weekly for about 4 weeks in the second dosing cycle;
subsequently, administering to the patient 8.7 mg-9.3 mg of the target compound weekly for at least 16 weeks in the third dosing cycle.

Further still, in some other specific embodiments, the method for preventing or treating obesity or overweight described herein comprises administering a target compound in the dosing regimen II as shown below:
administering to the patient 3 mg of the target compound weekly for about 4 weeks in the first dosing cycle;
subsequently, administering to the patient 6 mg of the target compound weekly for about 4 weeks in the second dosing cycle;
subsequently, administering to the patient 9 mg of the target compound weekly for at least 16 weeks in the third dosing cycle.

Even further, in some other specific embodiments, the method for preventing or treating obesity or overweight described herein comprises administering a target compound in the dosing regimen II as shown below:
administering to the patient 3 mg of the target compound once weekly for about 4 weeks in the first dosing cycle;
subsequently, administering to the patient 6 mg of the target compound once weekly for about 4 weeks in the second dosing cycle;
subsequently, administering to the patient 9 mg of the target compound once weekly for at least 16 weeks in the third dosing cycle.

In some other specific embodiments, the method for preventing or treating obesity or overweight described herein comprises administering a target compound in the dosing regimen II as shown below:
administering to the patient 2.5 mg-3.5 mg of the target compound weekly for 3-5 weeks in the first dosing cycle;
subsequently, administering to the patient 5.5 mg-6.5 mg of the target compound weekly for 3-5 weeks in the second dosing cycle;
subsequently, administering to the patient 8.5 mg-9.5 mg of the target compound weekly for at least 40 weeks in the third dosing cycle.

Further, in some other specific embodiments, the method for preventing or treating obesity or overweight described herein comprises administering a target compound in the dosing regimen II as shown below:
administering to the patient 2.7 mg-3.3 mg of the target compound weekly for about 4 weeks in the first dosing cycle;
subsequently, administering to the patient 5.7 mg-6.3 mg of the target compound weekly for about 4 weeks in the second dosing cycle;
subsequently, administering to the patient 8.7 mg-9.3 mg of the target compound weekly for at least 40 weeks in the third dosing cycle.

Further still, in some other specific embodiments, the method for preventing or treating obesity or overweight described herein comprises administering a target compound in the dosing regimen II as shown below:
administering to the patient 3 mg of the target compound weekly for about 4 weeks in the first dosing cycle;
subsequently, administering to the patient 6 mg of the target compound weekly for about 4 weeks in the second dosing cycle;
subsequently, administering to the patient 9 mg of the target compound weekly for at least 40 weeks in the third dosing cycle.

Even further, in some other specific embodiments, the method for preventing or treating obesity or overweight described herein comprises administering a target compound in the dosing regimen II as shown below:
administering to the patient 3 mg of the target compound once weekly for about 4 weeks in the first dosing cycle;
subsequently, administering to the patient 6 mg of the target compound once weekly for about 4 weeks in the second dosing cycle;
subsequently, administering to the patient 9 mg of the target compound once weekly for at least 40 weeks in the third dosing cycle.

### (Route of administration)

The specific route for administering the target compound to the subject in the present disclosure is not particularly limited. In some embodiments, the route of administration described herein, i.e., administration, is by injection. In some preferred embodiments, the administration described herein is subcutaneous injection.

### <Second aspect>

The present disclosure provides a method for improving body weight in a patient in need, comprising administering to the patient a prophylactically or therapeutically effective amount of a target compound in an dosing regimen consisting of at least three dosing cycles, wherein the at least three dosing cycles comprise:
(i) a first dosing cycle for at least 3 weeks of administering about one unit dose of the target compound weekly;
(ii) a second dosing cycle for at least 3 weeks of administering about two unit doses of the target compound weekly;
(iii) a third dosing cycle for at least 3 weeks of administering about three unit doses of the target compound weekly; and optionally,
(iv) a fourth dosing cycle for at least 3 weeks of administering about four unit doses of the target compound weekly,
wherein the unit dose is 2.2 mg-3.5 mg, and the target compound is selected from mazdutide or a pharmaceutically acceptable salt thereof.

The definitions of the duration of the dosing cycle, the number of administrations, the dose of administration, the dosing regimen, and the administration route therein are as described in <first aspect> of the present disclosure.

### <Third aspect>

The present disclosure provides a target compound for use in preventing or treating obesity or overweight in a patient in need, wherein a prophylactically or therapeutically effective amount of the target compound is administered to a patient in need in an dosing regimen consisting of at least three dosing cycles; the at least three dosing cycles comprise:
(i) a first dosing cycle for at least 3 weeks of administering about one unit dose of the target compound weekly;
(ii) a second dosing cycle for at least 3 weeks of administering about two unit doses of the target compound weekly;
(iii) a third dosing cycle for at least 3 weeks of administering about three unit doses of the target compound weekly; and optionally,
(iv) a fourth dosing cycle for at least 3 weeks of administering about four unit doses of the target compound weekly,
wherein the unit dose is 2.2 mg-3.5 mg, and the target compound is selected from mazdutide or a pharmaceutically acceptable salt thereof.

The definitions of the duration of the dosing cycle, the number of administrations, the dose of administration, the dosing regimen, and the administration route therein are as described in <first aspect> of the present disclosure.

### Examples

The present disclosure is further illustrated by the following examples, but any example or a combination thereof should not be construed as limiting the scope or embodiment of the present disclosure. The scope of the present disclosure is defined by the appended claims, and the scope defined by the claims will be clearly understood by those of ordinary skill in the art from this specification and the common general knowledge in the field. Any modification or change can be made to the technical solution of the present disclosure by those skilled in the art without departing from the spirit and scope of the present disclosure, and such modifications and changes are also included in the scope of the present disclosure.

The examples, in which specific conditions are not specified, were carried out according to conventional conditions or conditions recommended by the manufacturer. Reagents or instruments without specified manufacturers are conventional products that are commercially available. In the following detailed description, numerous specific details are set forth in order to provide a better understanding of the present disclosure. It should be understood by those skilled in the art that the present disclosure may be performed without some of these specific details. In other examples, methods, means, devices, and steps that are well known to those skilled in the art have not been described in detail so as not to obscure the present disclosure.

Unless otherwise defined, all technical and scientific terms used herein have the same meaning as those commonly understood by those of ordinary skill in the art. Unless otherwise indicated, all units used in this specification are international standard units, and the numerical values and numerical ranges appearing in the present disclosure should be understood to include unavoidable systematic errors.

### Example 1: Clinical Trial of Mazdutide Dosing Regimen

### I. Test drugs

The mazdutide formulation is mazdutide for injection consisting of 2 mg of mazdutide and inactive ingredients tris(hydroxymethyl)aminomethane, mannitol, and sucrose. The contents in the vial were reconstituted in sterile water for injection to give a clear solution of mazdutide.

The placebo was a mazdutide mimic, consisting of the inactive ingredients tris(hydroxymethyl)aminomethane, mannitol and sucrose. The contents in the vial were reconstituted in sterile water for injection to give a clear solution of the inactive ingredients.

The specification of the formulations was 2 mg/vial, and the placebo was in the same specification as the investigational formulation.

### II. Inclusion/Exclusion criteria for subjects

A) The main inclusion criteria are as follows:
   1. Aged 18-75 years (inclusive), male or female.
   2. For obesity, BMI ≥ 28.0 kg/m²; for overweight, 24 ≤ BMI < 28.0 kg/m² with at least one of the following: i, increased appetite, intolerable hunger before meals, and increased food consumption; ii, comorbidity with one or more of pre-diabetes (impaired fasting glucose and/or impaired glucose tolerance), hypertension, dyslipidemia, and fatty liver (within 6 months before the screening); iii, comorbidity with weight-bearing joint pain; iv, obesity-related dyspnea or obstructive sleep apnea syndrome.
   3. Weight change < 5% after at least 12 weeks of diet/exercise weight control alone at the time of screening.
B) Main exclusion criteria:
   1. Use of any of the following drugs or treatments before the screening:
      1) previous use of either GLP-1 receptor (GLP-1R) agonists or GLP-1R/GCGR agonists;
      2) use of drugs that may have an effect on body weight within 3 months before the screening, including: systemic steroid hormone therapy (intravenous, oral, or intraarticular administration), metformin, SGLT2 inhibitors, thiazolidinediones (TZD), tricyclic antidepressants, psychotropic agents, or sedatives (e.g., imipramine, amitriptyline, mirtazapine, paroxetine, phenelzine, chlorpromazine, thioridazine, clozapine, olanzapine, valproic acid, valproic acid derivatives, and lithium salts), and the like;
      3) use of Chinese herbal medicines or health care products that may have an effect on body weight within 3 months before the screening;
      4) previous or current use of weight-loss drugs within 3 months before the screening, such as: sibutramine hydrochloride, orlistat, phentermine, phenylpropanolamine, mazindol, phentermine, amfepramone, lorcaserin, compounded phentermine/topiramate, compounded naltrexone/amfepramone, etc.;
      5) participation in other clinical trials (reception of the test drug treatment) within 3 months before the screening.
   2. A history or evidence of any of the following diseases before the screening:
      1) confirmed diabetes according to WHO criteria 1999;
      2) fasting venous blood glucose ≥ 7.0 mmol/L at the time of screening or venous blood glucose ≥ 11.1 mmol/L at two hours after the sugar load of a 75 g oral glucose tolerance test (OGTT) (subjects with fasting blood glucose of 6.1-7.0 mmol/L at the time of screening were confirmed by the venous blood glucose at two hours after the sugar load of the OGTT);
      3) previous or current retinopathy at the time of screening;
      4) secondary diseases or drug-induced obesity, including: cortisol hormone elevation (e.g., Cushing's syndrome), obesity caused by pituitary and hypothalamus injury, obesity caused by reduction/withdrawal of weight-loss drugs, etc.;
      5) previous bariatric surgery, or acupuncture for weight loss, etc. within 1 year before the screening;
      6) a history of depression, or a history of serious psychiatric disorders, for example: schizophrenia, bipolar disorder, and the like;
      7) uncontrolled hypertension at the time of screening despite at least 4 weeks of hypotensive drug treatment, defined as: a systolic blood pressure of > 140 mmHg and/or a diastolic blood pressure of > 100 mmHg;
      8) a systolic blood pressure of < 90 mmHg and/or a diastolic blood pressure of < 50 mmHg at the time of screening;
      9) a history of malignancy (except for cured basal cell carcinoma and cervical carcinoma *in situ*) at the time of screening;
      10) cardiac diseases (such as angina pectoris, myocardial infarction, cardiomyopathy, acute and chronic heart failure, etc.) at the time of screening;
      11) hemorrhagic or ischemic stroke or transient ischemic attack within 6 months before the screening;
      12) a history or family history of medullary thyroid cancer, or MEN (multiple endocrine neoplasia) 2A or 2B syndrome at the time of screening;
      13) a history of acute or chronic pancreatitis, gallbladder disease and pancreas injury at the time of screening;
      14) chronic gastrointestinal diseases and systemic diseases that may affect gastrointestinal motility at the time of screening, or use of drugs that may change gastrointestinal motility, appetite or absorption within 3 months before the screening;
      15) limb deformity or defects that may affect the accurate measurement of indexes such as height, weight, and the like;
      16) ineligibility as determined by the investigator due to major and medium surgery, serious trauma, or serious infection within 1 month before the screening;
      17) previous suicidal tendency or suicidal behavior;
      18) scheduled surgery during the study, except for outpatient surgeries that do not affect the safety of the subject or the study results as determined by the investigator;
      19) human immunodeficiency virus (HIV) antibody, hepatitis B surface antigen (HBsAg), or hepatitis C virus (HCV) antibody, or syphilis antibody positive at the time of screening;
      20) a history of alcohol abuse within 1 month before the screening. Alcohol abuse is defined as: over 21 units for men or over 14 units for women weekly on average, or unwillingness to cease drinking within 24 hours before dosing and throughout the study period (one unit = 360 mL of beer, or 150 mL of wine, or 45 mL of distilled spirits);
      21) drug abuse and positivity in a urine test for drugs at the time of screening.
   3. Any one of the laboratory tests meets the following criteria (those of proper reasons at the time of screening can be re-examined within one week, and the reason for retesting should be documented by the investigator):
      1) serum calcitonin ≥ 15 ng/L at the time of screening;
      2) at the time of screening, alanine aminotransferase ≥ 2.0 × ULN and/or aspartate aminotransferase ≥ 2.0 × ULN and/or total bilirubin ≥ 1.0 × ULN and/or alkaline phosphatase ≥ 2.0 × ULN;
      3) glomerular filtration rate eGFR < 60 mL/min/1.73 m² at the time of screening, as estimated using the CKD-EPI equation;
      4) abnormal thyroid function (FT3, FT4, or TSH) at the time of screening;
      5) fasting triglyceride ≥ 5.64 mmol/L (500 mg/dL) at the time of screening; if the patient is receiving a lipid-regulating treatment, the dose of the treatment must be stable for 30 days before the screening;
      6) blood amylase or lipase > 2.0 × ULN at the time of screening;
      7) an international normalized ratio (INR) of prothrombin time greater than the upper limit of the normal range at the time of screening.
   4. Heart rate < 50 beats/min or > 90 beats/min as measured by 12-lead electrocardiogram (ECG) at the time of screening.
   5. Clinically significant abnormality in 12-lead ECG at the time of screening: atrioventricular block degree II or degree III, long QT syndrome or QTcF > 450 ms (see Attachment 3 for the formula), PR interval < 120 ms or PR interval > 220 ms, QRS > 120 ms, left or right bundle branch block, pre-excitation syndrome or severe arrhythmia requiring treatment in the absence of cardiac pacemaker.
   6. Pregnant or lactating women, or men or women with fertility unwilling to take contraceptive measures throughout the study period.
   7. Blood donation and/or blood loss of ≥ 400 mL or bone marrow donation within 3 months before the screening, or the presence of hemoglobinopathy, hemolytic anemia, sickle-cell anemia, or hemoglobin < 110 g/L (male) or < 100 g/L (female).
   8. Ineligibility due to any other factors that might affect the efficacy or safety assessment of the study as determined by the investigator.

### III. Dosing regimen and process

There were 5 cohorts in the study. Subjects in each cohort were randomized in a 2:1 ratio into the mazdutide treatment group (n = 8) and the placebo group (n = 4). The subcutaneous injection dosing regimens for mazdutide or placebo in cohort 1, cohort 2, cohort 3, cohort 4, and cohort 5 are described below:
Specification: The placebo was in the same specification as the investigational formulation;
Route of administration: subcutaneous injection, once weekly;
Dosing regimen:
   Cohort 1 (denoted as 3 mg cohort): 1.0 mg for 4 weeks + 2.0 mg for 4 weeks + 3.0 mg for 4 weeks;
   Cohort 2 (denoted as 4.5 mg cohort): 1.5 mg for 4 weeks + 3.0 mg for 4 weeks + 4.5 mg for 4 weeks;
   Cohort 3 (denoted as 6 mg cohort): 2.0 mg for 4 weeks + 4.0 mg for 4 weeks + 6.0 mg for 4 weeks;
   Cohort 4 (denoted as 10 mg cohort): 2.5 mg for 4 weeks + 5.0 mg for 4 weeks + 7.5 mg for 4 weeks + 10.0 mg for 4 weeks;
   Cohort 5 (denoted as 9 mg cohort): 3.0 mg for 4 weeks + 6.0 mg for 4 weeks + 9.0 mg for 4 weeks.

### IV. Experimental results

There were 5 cohorts in the study, and mazdutide showed significant weight loss efficacy in different doses.

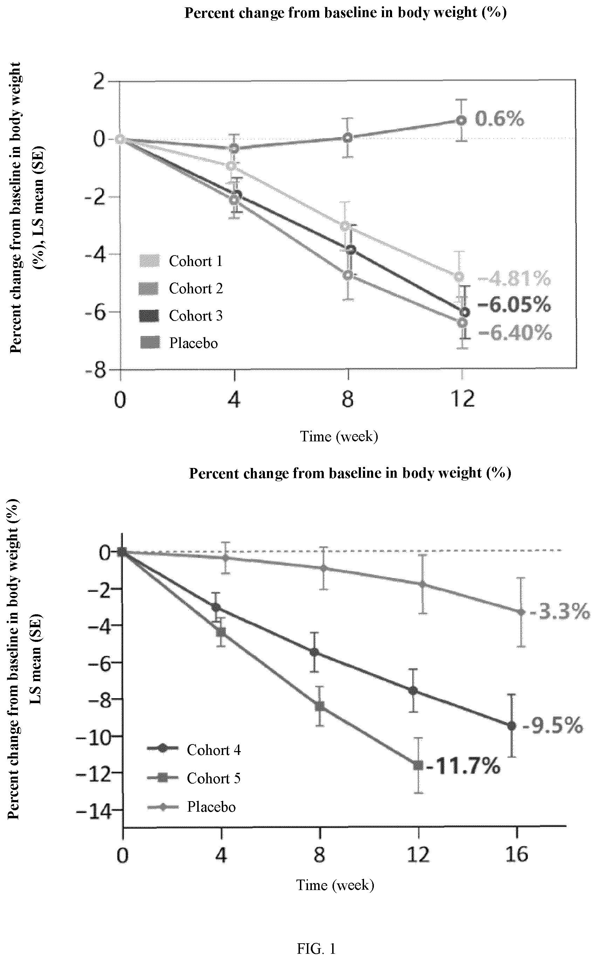

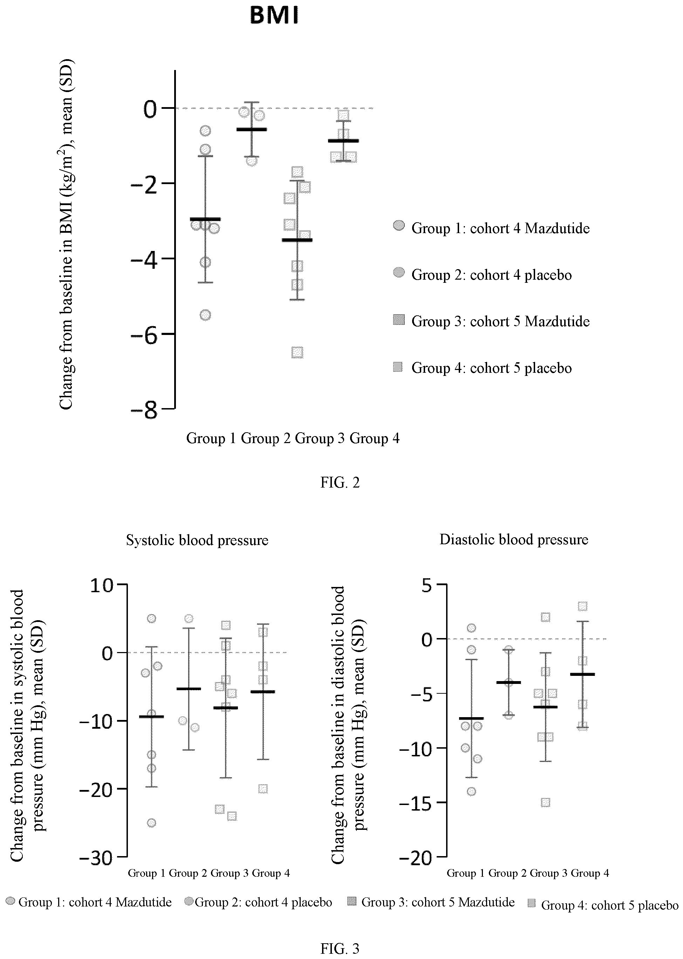

After 12 weeks of administration, the least squares means of percent change from baseline in body weight of subjects in the mazdutide group in cohort 1 (1 mg-2 mg-3 mg), cohort 2 (1.5 mg-3 mg-4.5 mg), cohort 3 (2 mg-4 mg-6 mg), and cohort 5 (3 mg-6 mg-9 mg) were -4.81%, -6.40%, -6.05%, and -11.7%, respectively; after 16 weeks of administration, the least squares mean of percent change from baseline in body weight of subjects in the mazdutide group in cohort 4 (2.5 mg-5 mg-7.5 mg-10 mg) was -9.5%. The results described above are shown in FIG. 1. The mean change results in BMI from baseline of subjects in cohort 4 and cohort 5 are shown in FIG. 2.

It can be seen that each dose group exhibited good weight loss efficacy in the subjects, and the weight loss effect did not reach a plateau after 12 weeks or 16 weeks of continuous administration, and the weight loss effect of the dosing regimen of cohort 5 of the present disclosure was particularly prominent in the patients. In addition, the previous research shows that the weight loss efficacy of GLP-1 medicaments is dose-dependent, but by comparing the dosing regimens of cohort 4 and cohort 5 in the present disclosure, the dose in the dosing regimen of cohort 5 was lower, and the treatment course was shorter compared with those of cohort 4, so that cohort 5 was proved to bring better weight loss efficacy not through the increase of the dose and the time, but reach the optimal weight loss efficacy in lower dose and shorter time through a specific dosing regimen, break through the cognition of the dosing regimen on GLP-1 medicaments in the prior art, and have important clinical application prospects.

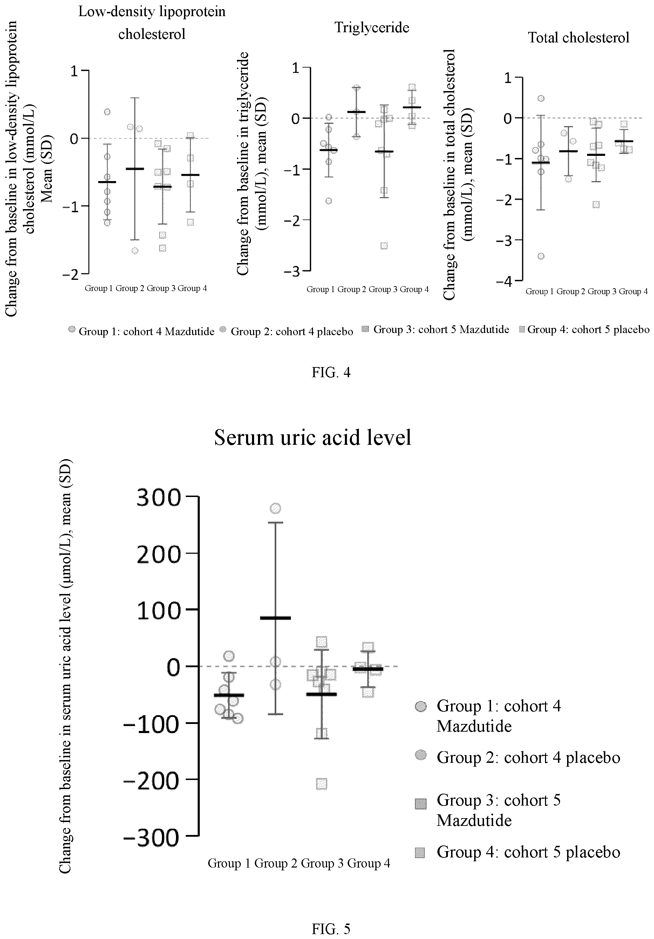

Meanwhile, on the basis of weight loss efficacy, different doses of mazdutide in 5 cohorts of the present disclosure also showed good beneficial effects in improving indexes such as blood pressure, blood lipid, and serum uric acid levels. The statistical results of the mean changes from baseline in blood pressure, blood lipid, and serum uric acid levels of the subjects in cohort 4 and cohort 5 of the present disclosure are shown in FIGs. 3-5.

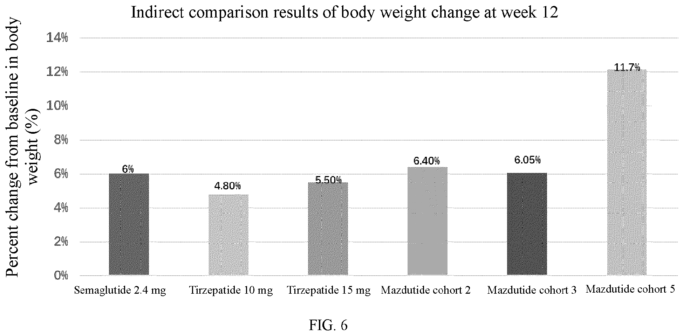

In addition, patient weight loss after 12 weeks of dosing regimens of other similar drugs with different doses was compared, such as the 2.4 mg Semaglutide dosing regimen (subcutaneous injection of Semaglutide, once weekly, 2.4 mg each time; see Khoo TK, Lin J. Once-Weekly Semaglutide in Adults with Overweight or Obesity. N Engl J Med. 2021 Jul 1;385(1):e4.) and the 10 mg or 15 mg Tirzepatide dosing regimen (subcutaneous injection of Tirzpatide, starting with a 2.5 mg dose, once weekly, a 2.5 mg increase in dose every 4 weeks until 10 mg or 15 mg; see Frias JP, Davies MJ, Rosenstock J, Pérez Manghi FC, Fernández Landó L, Bergman BK, Liu B, Cui X, Brown K; SURPASS-2 Investigators. Tirzepatide versus Semaglutide Once Weekly in Patients with Type 2 Diabetes. N Engl J Med. 2021 Aug 5;385(6):503-515.), and it was found that the mazdutide dosing regimen of cohort 5 of the present disclosure still exhibited a particularly pronounced weight loss effect in the subjects. The results described above are shown in FIG.6.

Safety results: None of the 5 cohorts had study drug dose adjustments during the study. Most treatment emergent adverse events (TEAEs) were mild, no severe TEAEs were reported, no TEAEs resulting in permanent drug withdrawal occurred, no treatment emergent serious adverse events (TESAEs) occurred, and no severe hypoglycemic events occurred. The overall tolerability and safety for the patients during the administration are proved to be good, and the dosing regimen provided by the present disclosure has high safety for clinical administration.

### Example 2: Further Clinical Trial of Mazdutide Dosing Regimen

### I. Test drugs

The same as in Example 1.

### II. Inclusion/Exclusion criteria for subjects

A) The main inclusion criteria are as follows:
   1. Aged 18-75 years (inclusive), male or female.
   2. For obesity, BMI ≥ 30.0 kg/m².
   3. The change of the body weight of the subject before and after the introduction period is less than 5.0%, and the formula is: percent body weight change = |(pre-random body weight - body weight on day of introduction)/pre-random body weight| × 100%.
   4. Capable of understanding the procedures and methods of the study, and willing to comply with the clinical protocol strictly to complete the study and to provide informed consent.
B) Main exclusion criteria:
   1. Suspected allergy to the study drugs or components or similar drugs by the investigator.
   2. Weight change > 5.0% after at least 12 weeks of diet/exercise weight control alone before the screening (chief complaint).
   3. Use of any of the following drugs or treatments before the screening:
      1) use of GLP-1 receptor (GLP-1R) agonist or GLP-1R/GCGR agonist or GIPR/GLP-1R agonist or GIPR/GLP-1R/GCGR agonist within 3 months before the screening;
      2) use of drugs that may have an effect on body weight within 3 months before the screening, including: systemic steroid hormone therapy (intravenous, oral, or intraarticular administration), tricyclic antidepressants, psychotropic agents, or sedatives (e.g., imipramine, amitriptyline, mirtazapine, paroxetine, phenelzine, chlorpromazine, thioridazine, clozapine, olanzapine, valproic acid, valproic acid derivatives, and lithium salts), and the like;
      3) use of Chinese herbal medicines, health care products, meal replacement, or the like, that may have an effect on body weight within 3 months before the screening;
      4) previous or current use of weight-loss drugs within 3 months before the screening, such as: sibutramine hydrochloride, orlistat, phentermine, phenylpropanolamine, mazindol, phentermine, amfepramone, lorcaserin, compounded phentermine/topiramate, compounded naltrexone/amfepramone, etc.;
      5) use of hypoglycemic drugs such as metformin, SGLT2 inhibitors, thiazolidinediones (TZD), and the like within 3 months before the screening;
      6) participation in other clinical trials (reception of the test drug treatment) within 3 months before the screening.
   4. A history or evidence of any of the following diseases:
      1) HbA1c ≥ 6.5% at the time of screening or previously confirmed type I or type II diabetes;
      2) fasting venous blood glucose ≥ 7.0 mmol/L at the time of screening or venous blood glucose ≥ 11.1 mmol/L at two hours after the sugar load of a 75 g oral glucose tolerance test (OGTT) (subjects with fasting blood glucose of 6.1-6.9 mmol/L at the time of screening were confirmed by the venous blood glucose at two hours after the sugar load of the OGTT);
      3) previous or current retinopathy at the time of screening;
      4) previous severe hypoglycemia or repeated symptomatic hypoglycemia (≥ 2 times within half a year);
      5) secondary diseases or drug-induced obesity, including: cortisol hormone elevation (e.g., Cushing's syndrome), obesity caused by pituitary and hypothalamus injury, obesity caused by reduction/withdrawal of weight-loss drugs, etc.;
      6) previous bariatric surgery (except for acupuncture for weight loss, liposuction, and abdominal fat-removing operation 1 year before the screening);
      7) planned bariatric surgery, acupuncture for weight loss, liposuction, abdominal fat-removing operation, and the like during the study;
      8) a history of moderate and severe depression, or a history of serious psychiatric disorders, for example: schizophrenia, bipolar disorder, and the like;
      9) previous suicidal tendency or suicidal behavior;
      10) PHQ (depression screening scale) questionnaire ≥ 15 points at the time of screening or at random;
      11) C-SSRS (Columbia-Suicide Severity Rating Scale) questionnaire category 4 or 5 at the time of screening or at random;
      12) uncontrolled hypertension within one month before the screening, defined as: a systolic blood pressure of ≥ 160 mmHg and/or a diastolic blood pressure of ≥ 100 mmHg (if a hypotensive drug is used, the hypertension needs to be stable for 1 month);
      13) previous or current malignancy (except for cured basal cell carcinoma and cervical carcinoma *in situ*) at the time of screening;
      14) a history of myocardial infarction, angina, acute and chronic heart failure, and cardiomyopathy, or a history of heart operations such as percutaneous coronary artery interventional therapy and coronary artery bypass transplantation, or obviously abnormal cardiac function indicated by echocardiography, and ineligibility as evaluated by the investigator;
      15) hemorrhagic or ischemic stroke or transient ischemic attack within 6 months before the screening;
      16) a history or family history of medullary thyroid cancer, or MEN (multiple endocrine neoplasia) 2A or 2B;
      17) a history of acute or chronic pancreatitis, gallbladder disease and pancreas injury;
      18) limb deformity or defects that may affect the accurate measurement of indexes such as height, weight, and the like;
      19) ineligibility as determined by the investigator due to major and medium surgery, serious trauma, or serious infection within 1 month before the screening;
      20) scheduled surgery during the study, except for outpatient surgeries that do not affect the safety of the subject or the study results as determined by the investigator;
      21) human immunodeficiency virus (HIV) antibody or hepatitis C virus (HCV) antibody, or syphilis antibody positive at the time of screening;
      22) positive hepatitis B surface antigen (HBsAg) and hepatitis B virus DNA ≥ 1000 IU/mL (subjects with the use of a medicament for resisting hepatitis B virus at the time of screening can not be enrolled);
      23) a history of alcohol and drug abuse at the time of screening. Alcohol abuse is defined as: over 21 units for men or over 14 units for women weekly on average, or unwillingness to cease drinking within 24 hours before dosing and throughout the study period (one unit = 360 mL of beer, or 150 mL of wine, or 45 mL of distilled spirits).
   5. Any one of the laboratory tests meets the following criteria at the time of screening (those of proper reasons can be re-examined within one week, and the reason for retesting should be documented by the investigator):
      1) serum calcitonin ≥ 20 ng/L (pg/mL);
      2) alanine aminotransferase ≥ 3.0 × upper limit of normal value (ULN) and/or aspartate aminotransferase ≥ 3.0 × ULN and/or total bilirubin ≥ 1.5 × ULN;
      3) glomerular filtration rate eGFR < 60 mL/min/1.73 m², as estimated using the CKD-EPI equation;
      4) abnormal thyroid function (TSH > 6 mIU/L or < 0.4 mIU/L);
      5) fasting triglyceride ≥ 5.64 mmol/L (500 mg/dL);
      6) blood amylase or lipase > 2.0 × ULN;
      7) an international normalized ratio (INR) of prothrombin time greater than the upper limit of the normal range;
      8) hemoglobin < 110 g/L (male) or < 100 g/L (female).
   6. Heart rate < 50 beats/min or > 100 beats/min as measured by 12-lead electrocardiogram (ECG) at the time of screening.
   7. Clinically significant abnormality in 12-lead ECG at the time of screening: atrioventricular block degree II or degree III, long QT syndrome or QTcF > 500 ms (see Appendix 4 for the formula), left or right bundle branch block, pre-excitation syndrome, or other significant arrhythmias (except for sinus arrhythmia).
   8. Pregnant or lactating women, or men or women with fertility unwilling to take contraceptive measures throughout the study period.
   9. Blood donation and/or blood loss of ≥ 400 mL or bone marrow donation within 3 months before the screening, or the presence of hemoglobinopathy, hemolytic anemia, and sickle-cell anemia.
   10. Ineligibility due to any other factors that might affect the efficacy or safety assessment of the study as determined by the investigator.

### III. Dosing regimen and process

Approximately 80 subjects were enrolled as planned for this study, and eligible subjects received placebo introduction for 2 weeks. After successful introduction, the subjects were randomly grouped according to the ratio of mazdutide:placebo = 3:1 and dosed under the dosing regimen. After the administration of the subjects was complete, a 12-week drug withdrawal follow-up may be performed.
Specification: The placebo was in the same specification as the investigational formulation;
Route of administration: subcutaneous injection, once weekly;
Dosing regimen: 3.0 mg for 4 weeks + 6.0 mg for 4 weeks + 9.0 mg for 16 weeks or 40 weeks.

### IV. Experimental results

80 subjects were enrolled for this study stage, and eligible subjects received placebo introduction for 2 weeks. After successful introduction, the subjects were randomly grouped into the mazdutide group or the placebo group. Administration mode: 3.0 mg QW for 4 weeks + 6.0 mg QW for 4 weeks + 9.0 mg QW for 16 weeks. After reaching the 24-week primary endpoint, the subjects may choose to have 24 weeks of extended treatment. The current study has reached a primary endpoint and the results show that the percentage change from baseline in body weight for the mazdutide group and placebo group was -13.25% and 2.11%, respectively, after 24 consecutive weeks of administration. The difference in percent change from baseline in body weight for the mazdutide group after 24 consecutive weeks of administration was -15.36% (p value less than 0.0001) compared with the placebo group. The changes from baseline in body weight for the mazdutide group and placebo group were -12.55 kg and 2.12 kg, respectively. The difference in change from baseline in body weight for the mazdutide group after 24 consecutive weeks of administration was -14.67 kg (p value less than 0.0001) compared with the placebo group. After 24 consecutive weeks of administration, 81.7%, 65.0%, 31.7%, and 21.7% of subjects in the mazdutide group had reduced body weight by at least 5%, 10%, 15%, and 20% from baseline, respectively, while none of the subjects had reduced body weight by 5% or more in the placebo group.

It can be seen that the weight loss efficacy in obese subjects can be further improved by increasing the administration time of the maintenance dose (e.g., 9.0 mg of maintenance dose administered consecutively for 16 weeks or 40 weeks, etc.) as compared to the cohort 5 dosing regimen of Example 1 (12 weeks of consecutive administration).

In terms of safety, until now, the mazdutide treatment group has good overall tolerability and safety, the dropout rate of the treatment group has been lower than that of the placebo group overall, no subject in the treatment group has terminated treatment in advance due to adverse events, and no serious adverse event has occurred. The most common treatment emergent adverse events, with the exception of COVID-19 infection, are gastrointestinal-related adverse events, and most are mild or moderate and are transient. Thus, the dosing regimen provided by the present disclosure has high safety for clinical administration.

## Claims

1. A method for preventing or treating obesity or overweight, comprising administering to a patient in need a prophylactically or therapeutically effective amount of a target compound in a dosing regimen consisting of at least three dosing cycles, wherein the at least three dosing cycles comprise:
(i) a first dosing cycle for at least 3 weeks of administering about one unit dose of the target compound weekly;
(ii) a second dosing cycle for at least 3 weeks of administering about two unit doses of the target compound weekly;
(iii) a third dosing cycle for at least 3 weeks of administering about three unit doses of the target compound weekly; and optionally,
(iv) a fourth dosing cycle for at least 3 weeks of administering about four unit doses of the target compound weekly,
wherein the unit dose is 2.2 mg-3.5 mg, and the target compound is selected from mazdutide or a pharmaceutically acceptable salt thereof.

2. The method according to claim 1, wherein the duration of the last dosing cycle in the dosing regimen is at least 3 weeks, preferably at least 4 weeks; the duration of each of the remaining dosing cycles is independently 3 weeks-5 weeks, preferably about 4 weeks;
optionally,
the third dosing cycle is taken as the last dosing cycle in the dosing regimen; moreover, the duration of the third dosing cycle is at least 3 weeks, preferably at least 4 weeks;
alternatively,
the fourth dosing cycle is taken as the last dosing cycle in the dosing regimen; moreover, the duration of the fourth dosing cycle is at least 3 weeks, preferably at least 4 weeks.

3. The method according to claim 1 or 2, wherein in each of the dosing cycles, the target compound is administered to the patient one or more times weekly;
preferably, in each of the dosing cycles, the target compound is administered to the patient once weekly.

4. The method according to any one of claims 1-3, wherein the unit dose is 2.2 mg-3 mg, 2.2 mg-2.8 mg, 2.4 mg-2.6 mg, 2.5 mg-3.5 mg, 2.7 mg-3.3 mg, or 2.9 mg-3.1 mg;
optionally, the unit dose is about 2.2 mg, about 2.3 mg, about 2.4 mg, about 2.5 mg, about 2.6 mg, about 2.7 mg, about 2.8 mg, about 2.9 mg, about 3.0 mg, about 3.1 mg, about 3.2 mg, about 3.3 mg, about 3.4 mg, or about 3.5 mg.

5. The method according to any one of claims 1-4, comprising administering the target compound in the dosing regimen I as shown below:
administering to the patient 2.2 mg-3 mg of the target compound weekly for 3-5 weeks in the first dosing cycle;
subsequently, administering to the patient 4.5 mg-5.5 mg of the target compound weekly for 3-5 weeks in the second dosing cycle;
subsequently, administering to the patient 7 mg-8 mg of the target compound weekly for 3-5 weeks in the third dosing cycle;
subsequently, administering to the patient 9.5 mg-10.5 mg of the target compound weekly for at least 3 weeks in the fourth dosing cycle;
optionally, in the first dosing cycle, administering to the patient 2.2 mg-2.8 mg of the target compound weekly for about 4 weeks; preferably administering to the patient about 2.5 mg of the target compound weekly for about 4 weeks; more preferably administering to the patient about 2.5 mg of the target compound once weekly for about 4 weeks;
optionally, in the second dosing cycle, administering to the patient 4.7 mg-5.3 mg of the target compound weekly for about 4 weeks; preferably administering to the patient about 5 mg of the target compound weekly for about 4 weeks; more preferably administering to the patient about 5 mg of the target compound once weekly for about 4 weeks;
optionally, in the third dosing cycle, administering to the patient 7.2 mg-7.8 mg of the target compound weekly for about 4 weeks; preferably administering to the patient about 7.5 mg of the target compound weekly for about 4 weeks; more preferably administering to the patient about 7.5 mg of the target compound once weekly for about 4 weeks;
optionally, in the fourth dosing cycle, administering to the patient 9.7 mg-10.3 mg of the target compound weekly for at least 4 weeks; preferably administering to the patient about 10 mg of the target compound weekly for at least 4 weeks; more preferably administering to the patient about 10 mg of the target compound once weekly for at least 4 weeks.

6. The method according to any one of claims 1-4, comprising administering the target compound in the dosing regimen II as shown below:
administering to the patient 2.5 mg-3.5 mg of the target compound weekly for 3-5 weeks in the first dosing cycle;
subsequently, administering to the patient 5.5 mg-6.5 mg of the target compound weekly for 3-5 weeks in the second dosing cycle;
subsequently, administering to the patient 8.5 mg-9.5 mg of the target compound weekly for at least 3 weeks in the third dosing cycle;
optionally, in the first dosing cycle, administering to the patient 2.7 mg-3.3 mg of the target compound weekly for about 4 weeks; preferably administering to the patient about 3 mg of the target compound weekly for about 4 weeks; more preferably administering to the patient about 3 mg of the target compound once weekly for about 4 weeks;
optionally, in the second dosing cycle, administering to the patient 5.7 mg-6.3 mg of the target compound weekly for about 4 weeks; preferably administering to the patient about 6 mg of the target compound weekly for about 4 weeks; more preferably administering to the patient about 6 mg of the target compound once weekly for about 4 weeks;
optionally, in the third dosing cycle, administering to the patient 8.7 mg-9.3 mg of the target compound weekly for at least 4 weeks; preferably administering to the patient about 9 mg of the target compound weekly for at least 4 weeks; more preferably administering to the patient about 9 mg of the target compound once weekly for at least 4 weeks;
optionally, in the third dosing cycle, administering to the patient 8.7 mg-9.3 mg of the target compound weekly for at least 16 weeks; preferably administering to the patient about 9 mg of the target compound weekly for at least 16 weeks; more preferably administering to the patient about 9 mg of the target compound once weekly for at least 16 weeks;
optionally, in the third dosing cycle, administering to the patient 8.7 mg-9.3 mg of the target compound weekly for at least 40 weeks; preferably administering to the patient about 9 mg of the target compound weekly for at least 40 weeks; more preferably administering to the patient about 9 mg of the target compound once weekly for at least 40 weeks.

7. The method according to any one of claims 1-6, wherein the administration is by injection, preferably subcutaneous injection.

8. A method for improving body weight in a patient in need, comprising administering to the patient a prophylactically or therapeutically effective amount of a target compound in an dosing regimen consisting of at least three dosing cycles, wherein the at least three dosing cycles comprise:
(i) a first dosing cycle for at least 3 weeks of administering about one unit dose of the target compound weekly;
(ii) a second dosing cycle for at least 3 weeks of administering about two unit doses of the target compound weekly;
(iii) a third dosing cycle for at least 3 weeks of administering about three unit doses of the target compound weekly; and optionally,
(iv) a fourth dosing cycle for at least 3 weeks of administering about four unit doses of the target compound weekly,
wherein the unit dose is 2.2 mg-3.5 mg, and the target compound is selected from mazdutide or a pharmaceutically acceptable salt thereof;
preferably, a prophylactically or therapeutically effective amount of the target compound is administered to the patient using the method according to any one of claims 1-7.

9. A target compound for use in preventing or treating obesity or overweight in a patient in need, wherein a prophylactically or therapeutically effective amount of the target compound is administered to a patient in need in an dosing regimen consisting of at least three dosing cycles; the at least three dosing cycles comprise:
(i) a first dosing cycle for at least 3 weeks of administering about one unit dose of the target compound weekly;
(ii) a second dosing cycle for at least 3 weeks of administering about two unit doses of the target compound weekly;
(iii) a third dosing cycle for at least 3 weeks of administering about three unit doses of the target compound weekly; and optionally,
(iv) a fourth dosing cycle for at least 3 weeks of administering about four unit doses of the target compound weekly,
wherein the unit dose is 2.2 mg-3.5 mg, and the target compound is selected from mazdutide or a pharmaceutically acceptable salt thereof;
preferably, a prophylactically or therapeutically effective amount of the target compound is administered to the patient using the method according to any one of claims 1-7.
